# EUROPEAN PATENT APPLICATION

(11) **EP 1 533 741 A2**
(43) Date of publication of application: **25.05.2005**
(21) Application number: 04255864.3
(22) Date of filing: 24.09.2004
(51) Int. Cl.: G06F 19/00

(54) **Collection and analysis of procedural information**

(30) Priority: 24.09.2003 US 670847
(71) Applicant: Pacesetter, Inc., Sylmar, CA 91342-9221 (US)
(72) Inventor: Siew, Er, Fair Oaks Ranch CA 91387 (US)
(74) Representative: Rees, David Christopher

(57) **Abstract**

An exemplary method includes recording procedures performed by a care provider during a follow-up consultation with a patient having an implanted device, analyzing the procedures, and recommending one or more procedures for a subsequent follow-up consultation. An exemplary programming system for an implanted device includes an input for inputting procedural information, statistical analysis software for analyzing the procedural information, and an output for outputting one or more recommended procedures based at least in part on a statistical analysis of the procedural information performed using the statistical analysis software. Other exemplary methods, devices and/or systems are also disclosed.

## Description

Exemplary methods and/or devices presented herein generally relate to patient's having implanted devices for cardiac pacing and/or stimulation therapies. Various exemplary methods and/or devices concern collection and/or analysis of procedural information related to such therapies.

Over the past several decades thousands of patients have been fitted with implantable cardiac pacing, cardioversion, defibrillation and/or other devices that can delivery cardiac therapy. Monitoring of such patients often involves a collaborative effort of physicians, nurses, device manufactures, regulatory agencies, insurance companies and health care institution managers. Patients may receive care through in-person office visits, telephone consultations or other communication routes (e.g., Internet, etc.). Care is often scheduled on a periodic basis wherein office visits may occur less frequently than distance consultations (e.g., typically at least four times per year). During an office or a distance consultation, automated analyses may be performed wherein an implanted device communicates with a receiver and/or transmitter unit, which is typically connected to a computer. Aside from such procedures, little else is automated. For example, care providers typically need to follow extensive written guidelines as to how to assess a patient's condition and/or condition of an implanted device (e.g., follow-up guidelines). Further, information collected by a care provider typically requires data entry during and/or after a consultation. Yet further, a change in care provider or, for example, a lengthy delay between consultations may require a searching of patient or other records in an effort to reconstruct a consultation procedure history, which can increase demands on a health care system. In an emergency situation, lack of such a history may impact treatment and/or outcome. While data related to follow-up is typically stored, lack of a procedural history may make the data less useful, especially where time is a factor. Hence, a need exists for methods and/or devices that allow for collection and/or analysis of procedure information and, in particular, information for care provider procedures.

As described herein, various exemplary methods, systems and/or devices aim to collect and/or analyze procedure information and/or increase automation of procedures related to care providers that care for patients having implanted cardiac therapy devices. Such exemplary methods, systems and/or devices can reduce demands on a health care system, allow for more efficient consultations and/or increase knowledge of procedures related to providing care. Exemplary methods, systems and/or devices having these and/or other advantages are also disclosed.

An exemplary method includes recording procedures performed by a care provider during a follow-up consultation with a patient having an implanted device, analyzing the procedures, and recommending one or more procedures for a subsequent follow-up consultation. An exemplary programming system for an implanted device includes an input for inputting procedural information, statistical analysis software for analyzing the procedural information, and an output for outputting one or more recommended procedures based at least in part on a statistical analysis of the procedural information performed using the statistical analysis software. Other exemplary methods, devices and/or systems are also disclosed.

Features and advantages of the described implementations can be more readily understood by reference to the following description taken in conjunction with the accompanying drawings.
Fig. 1 is a simplified diagram illustrating an exemplary implantable stimulation device in electrical communication with at least three leads implanted into a patient's heart and at least one other lead for sensing and/or delivering stimulation and/or shock therapy.
Fig. 2 is a functional block diagram of an exemplary implantable stimulation device illustrating basic elements that are configured to provide cardioversion, defibrillation, pacing stimulation and/or autonomic nerve stimulation or other tissue and/or nerve stimulation. The implantable stimulation device is further configured to sense information and administer stimulation pulses responsive to such information.
Fig. 3 is a block diagram of various exemplary phases of procedures associated with a patient to have or having an implanted device.
Fig. 4 is a block diagram of exemplary follow-up procedures for a patient having an implanted device.
Fig. 5 is a diagram of an exemplary environment of implanted device manufacturers, patients and care providers.
Fig. 6 is a diagram of exemplary consultation cycles for patients having implanted devices.
Fig. 7 is a block diagram of an exemplary procedural information system.
Fig. 8 is a block diagram of an exemplary procedural record.
Fig. 9 is a block diagram of exemplary analyses for analyzing procedural and/or other information.
Fig. 10 is a block diagram of an exemplary method capable of execution on a programming system for an implanted device.
Fig. 11 is a diagram of an exemplary programming system capable of implementing various exemplary methods.

### Exemplary Stimulation Device

The techniques described below are intended to be implemented in connection with any stimulation device that is configured or configurable to stimulate nerves and/or stimulate and/or shock a patient's heart. Other implantable devices may be suitably used as well, for example, devices that sense, detect and/or collect information yet do not have stimulation capabilities or such capabilities implemented.

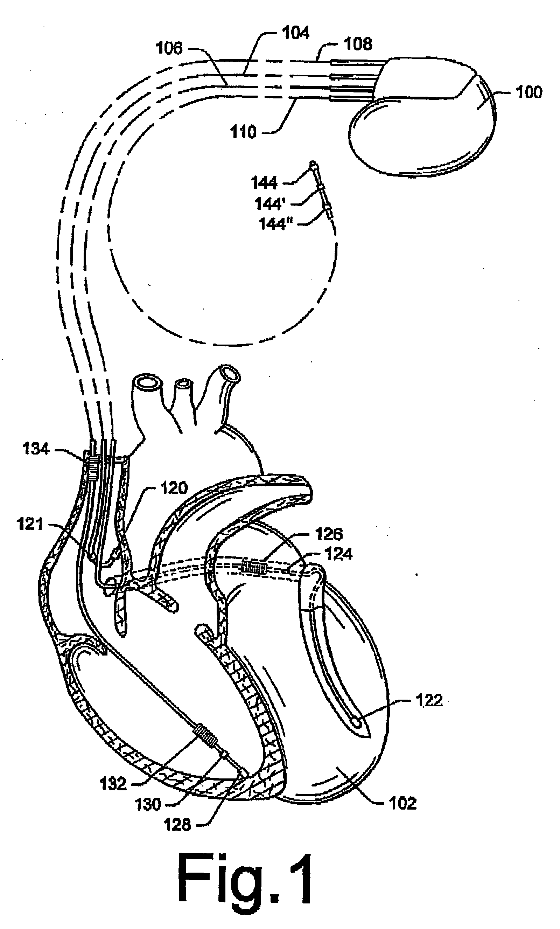

Fig. 1 shows an exemplary stimulation device 100 in electrical communication with a patient's heart 102 by way of three leads 104, 106, 108, suitable for delivering multi-chamber stimulation and shock therapy. The leads 104, 106, 108 are optionally configurable for delivery of stimulation pulses suitable for stimulation of autonomic nerves. In addition, the device 100 includes a fourth lead 110 having, in this implementation, three electrodes 144, 144', 144" suitable for stimulation of autonomic nerves and/or detection of other physiologic signals that may be used by the implanted system to modify the pacing parameters. This lead may be positioned in and/or near a patient's heart or near an autonomic nerve within a patient's body and remote from the heart. The right atrial lead 104, as the name implies, is positioned in and/or passes through a patient's right atrium. The right atrial lead 104 optionally senses atrial cardiac signals and/or provide right atrial chamber stimulation therapy. As shown in Fig. 1, the stimulation device 100 is coupled to an implantable right atrial lead 104 having, for example, an atrial tip electrode 120, which typically is implanted in the patient's right atrial appendage. The lead 104, as shown in Fig. 1, also includes an atrial ring electrode 121. Of course, the lead 104 may have other electrodes as well. For example, the right atrial lead optionally includes a distal bifurcation having electrodes suitable for stimulation of autonomic nerves.

To sense atrial cardiac signals, ventricular cardiac signals and/or to provide chamber pacing therapy, particularly on the left side of a patient's heart, the stimulation device 100 is coupled to a coronary sinus lead 106 designed for placement in the coronary sinus and/or tributary veins of the coronary sinus. Thus, the coronary sinus lead 106 is optionally suitable for positioning at least one distal electrode adjacent to the left ventricle and/or additional electrode(s) adjacent to the left atrium. In a normal heart, tributary veins of the coronary sinus include, but may not be limited to, the great cardiac vein, the left marginal vein, the left posterior ventricular vein, the middle cardiac vein, and the small cardiac vein.

Accordingly, an exemplary coronary sinus lead 106 is optionally designed to receive atrial and ventricular cardiac signals and to deliver left ventricular pacing therapy using, for example, at least a left ventricular tip electrode 122, left atrial pacing therapy using at least a left atrial ring electrode 124, and shocking therapy using at least a left atrial coil electrode 126. For a complete description of a coronary sinus lead, the reader is directed to U.S. Patent Application No. 09/457,277, filed 12/8/99, entitled "A Self-Anchoring, Steerable Coronary Sinus Lead" (Pianca et al.); and U.S. Patent No. 5,466,254, "Coronary Sinus Lead with Atrial Sensing Capability" (Helland). The coronary sinus lead 106 further optionally includes electrodes for stimulation of autonomic nerves. Such a lead may include pacing and autonomic nerve stimulation functionality and may further include bifurcations or legs. For example, an exemplary coronary sinus lead includes pacing electrodes capable of delivering pacing pulses to a patient's left ventricle and at least one electrode capable of stimulating an autonomic nerve. An exemplary coronary sinus lead (or left ventricular lead or left atrial lead) may also include at least one electrode capable of stimulating an autonomic nerve, such an electrode may be positioned on the lead or a bifurcation or leg of the lead.

Stimulation device 100 is also shown in electrical communication with the patient's heart 102 by way of an implantable right ventricular lead 108 having, in this exemplary implementation, a right ventricular tip electrode 128, a right ventricular ring electrode 130, a right ventricular (RV) coil electrode 132, and an SVC coil electrode 134. Typically, the right ventricular lead 108 is transvenously inserted into the heart 102 to place the right ventricular tip electrode 128 in the right ventricular apex so that the RV coil electrode 132 will be positioned in the right ventricle and the SVC coil electrode 134 will be positioned in the superior vena cava. Accordingly, the right ventricular lead 108 is capable of sensing or receiving cardiac signals, and delivering stimulation in the form of pacing and shock therapy to the right ventricle. An exemplary right ventricular lead may also include at least one electrode capable of stimulating an autonomic nerve, such an electrode may be positioned on the lead or a bifurcation or leg of the lead.

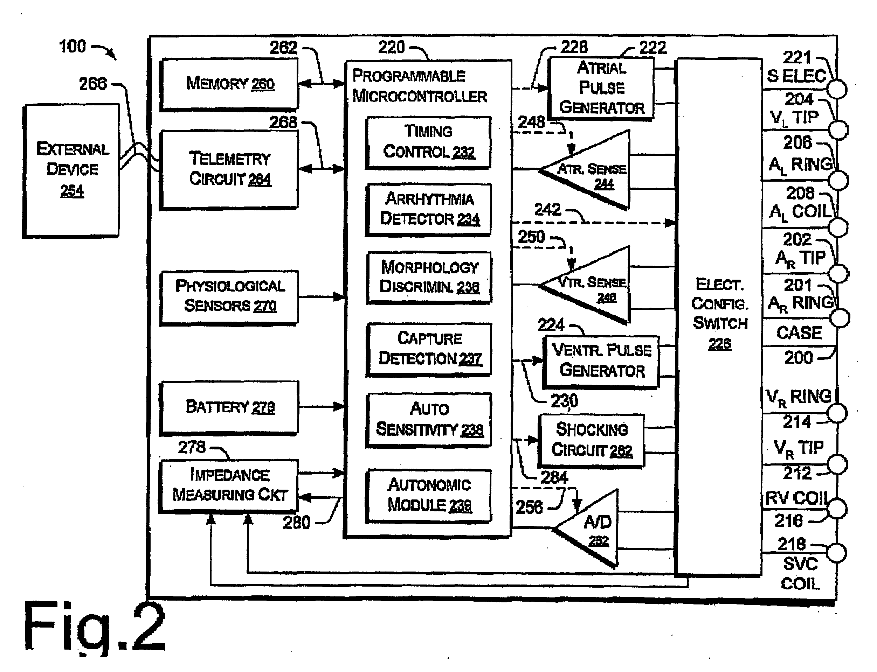

Fig. 2 shows an exemplary, simplified block diagram depicting various components of stimulation device 100. The stimulation device 100 can be capable of treating both fast and slow arrhythmias with stimulation therapy, including cardioversion, defibrillation, and pacing stimulation. The stimulation device can be solely or further capable of delivering stimuli to autonomic nerves. While a particular multi-chamber device is shown, it is to be appreciated and understood that this is done for illustration purposes only. Thus, the techniques and methods described below can be implemented in connection with any suitably configured or configurable stimulation device. Accordingly, one of skill in the art could readily duplicate, eliminate, or disable the appropriate circuitry in any desired combination to provide a device capable of treating the appropriate chamber(s) or regions of a patient's heart with cardioversion, defibrillation, pacing stimulation, and/or autonomic nerve stimulation.

Housing 200 for stimulation device 100 is often referred to as the "can", "case" or "case electrode", and may be programmably selected to act as the return electrode for all "unipolar" modes. Housing 200 may further be used as a return electrode alone or in combination with one or more of the coil electrodes 126, 132 and 134 for shocking purposes. Housing 200 further includes a connector (not shown) having a plurality of terminals 201, 202, 204, 206, 208, 212, 214, 216, 218, 221 (shown schematically and, for convenience, the names of the electrodes to which they are connected are shown next to the terminals).

To achieve right atrial sensing, pacing and/or autonomic stimulation, the connector includes at least a right atrial tip terminal (AR TIP) 202 adapted for connection to the atrial tip electrode 120. A right atrial ring terminal (AR RING) 201 is also shown, which is adapted for connection to the atrial ring electrode 121. To achieve left chamber sensing, pacing, shocking, and/or autonomic stimulation, the connector includes at least a left ventricular tip terminal (VL TIP) 204, a left atrial ring terminal (AL RING) 206, and a left atrial shocking terminal (AL COIL) 208, which are adapted for connection to the left ventricular tip electrode 122, the left atrial ring electrode 124, and the left atrial coil electrode 126, respectively. Connection to suitable autonomic nerve stimulation electrodes is also possible via these and/or other terminals (e.g., via a nerve stimulation terminal S ELEC 221).

To support right chamber sensing, pacing, shocking, and/or autonomic nerve stimulation, the connector further includes a right ventricular tip terminal (VR TIP) 212, a right ventricular ring terminal (VR RING) 214, a right ventricular shocking terminal (RV COIL) 216, and a superior vena cava shocking terminal (SVC COIL) 218, which are adapted for connection to the right ventricular tip electrode 128, right ventricular ring electrode 130, the RV coil electrode 132, and the SVC coil electrode 134, respectively. Connection to suitable autonomic nerve stimulation electrodes is also possible via these and/or other terminals (e.g., via the nerve stimulation terminal S ELEC 221).

At the core of the stimulation device 100 is a programmable microcontroller 220 that controls the various modes of stimulation therapy. As is well known in the art, microcontroller 220 typically includes a microprocessor, or equivalent control circuitry, designed specifically for controlling the delivery of stimulation therapy, and may further include RAM or ROM memory, logic and timing circuitry, state machine circuitry, and I/O circuitry. Typically, microcontroller 220 includes the ability to process or monitor input signals (data or information) as controlled by a program code stored in a designated block of memory. The type of microcontroller is not critical to the described implementations. Rather, any suitable microcontroller 220 may be used that carries out the functions described herein. The use of microprocessor-based control circuits for performing timing and data analysis functions are well known in the art.

Representative types of control circuitry that may be used in connection with the described embodiments can include the microprocessor-based control system of U.S. Patent No. 4,940,052 (Mann et al.), the state-machine of U.S. Patent Nos. 4,712,555 (Thornander et al.) and 4,944,298 (Sholder). For a more detailed description of the various timing intervals used within the stimulation device and their interrelationship, see U.S. Patent 4,788,980 (Mann et al.).

Fig. 2 also shows an atrial pulse generator 222 and a ventricular pulse generator 224 that generate pacing stimulation pulses for delivery by the right atrial lead 104, the coronary sinus lead 106, and/or the right ventricular lead 108 via an electrode configuration switch 226. It is understood that in order to provide stimulation therapy in each of the four chambers of the heart (or to autonomic nerves) the atrial and ventricular pulse generators, 222 and 224, may include dedicated, independent pulse generators, multiplexed pulse generators, or shared pulse generators. The pulse generators 222 and 224 are controlled by the microcontroller 220 via appropriate control signals 228 and 230, respectively, to trigger or inhibit the stimulation pulses.

Microcontroller 220 further includes timing control circuitry 232 to control the timing of the stimulation pulses (e.g., pacing rate, atrio-ventricular (AV) delay, interatrial conduction (A-A) delay, or interventricular conduction (V-V) delay, etc.) as well as to keep track of the timing of refractory periods, blanking intervals, noise detection windows, evoked response windows, alert intervals, marker channel timing, etc., which is well known in the art.

Microcontroller 220 further includes an arrhythmia detector 234 and optionally an orthostatic compensator and a minute ventilation (MV) response module, the latter is not shown in Fig. 2. These components can be utilized by the stimulation device 100 for determining desirable times to administer various therapies, including those to reduce the effects of orthostatic hypotension. The aforementioned components may be implemented in hardware as part of the microcontroller 220, or as software/firmware instructions programmed into the device and executed on the microcontroller 220 during certain modes of operation.

Microcontroller 220 further includes a morphology discrimination module 236, a capture detection module 237 and an auto sensing module 238. These modules are optionally used to implement various exemplary recognition algorithms and/or methods presented below. The aforementioned components may be implemented in hardware as part of the microcontroller 220, or as software/firmware instructions programmed into the device and executed on the microcontroller 220 during certain modes of operation.

Microcontroller 220 further includes an autonomic nerve stimulation module 239 for performing a variety of tasks related to autonomic nerve stimulation. This component can be utilized by the stimulation device 100 for determining desirable times to administer various therapies, including, but not limited to, parasympathetic stimulation. The autonomic module 239 may be implemented in hardware as part of the microcontroller 220, or as software/firmware instructions programmed into the device and executed on the microcontroller 220 during certain modes of operation.

The electronic configuration switch 226 includes a plurality of switches for connecting the desired electrodes to the appropriate I/O circuits, thereby providing complete electrode programmability. Accordingly, switch 226, in response to a control signal 242 from the microcontroller 220, determines the polarity of the stimulation pulses (e.g., unipolar, bipolar, etc.) by selectively closing the appropriate combination of switches (not shown) as is known in the art.

Atrial sensing circuits 244 and ventricular sensing circuits 246 may also be selectively coupled to the right atrial lead 104, coronary sinus lead 106, and the right ventricular lead 108, through the switch 226 for detecting the presence of cardiac activity in each of the four chambers of the heart. Accordingly, the atrial (ATR. SENSE) and ventricular (VTR. SENSE) sensing circuits, 244 and 246, may include dedicated sense amplifiers, multiplexed amplifiers, or shared amplifiers. Switch 226 determines the "sensing polarity" of the cardiac signal by selectively closing the appropriate switches, as is also known in the art. In this way, the clinician may program the sensing polarity independent of the stimulation polarity. The sensing circuits (e.g., 244 and 246) are optionally capable of obtaining information indicative of tissue capture.

Each sensing circuit 244 and 246 preferably employs one or more low power, precision amplifiers with programmable gain and/or automatic gain control, bandpass filtering, and a threshold detection circuit, as known in the art, to selectively sense the cardiac signal of interest. The automatic gain control enables the device 100 to deal effectively with the difficult problem of sensing the low amplitude signal characteristics of atrial or ventricular fibrillation.

The outputs of the atrial and ventricular sensing circuits 244 and 246 are connected to the microcontroller 220, which, in turn, is able to trigger or inhibit the atrial and ventricular pulse generators 222 and 224, respectively, in a demand fashion in response to the absence or presence of cardiac activity in the appropriate chambers of the heart. Furthermore, as described herein, the microcontroller 220 is also capable of analyzing information output from the sensing circuits 244 and 246 and/or the data acquisition system 252 to determine or detect whether and to what degree tissue capture has occurred and to program a pulse, or pulses, in response to such determinations. The sensing circuits 244 and 246, in turn, receive control signals over signal lines 248 and 250 from the microcontroller 220 for purposes of controlling the gain, threshold, polarization charge removal circuitry (not shown), and the timing of any blocking circuitry (not shown) coupled to the inputs of the sensing circuits, 244 and 246, as is known in the art.

For arrhythmia detection, the device 100 may utilize the atrial and ventricular sensing circuits, 244 and 246, to sense cardiac signals to determine whether a rhythm is physiologic or pathologic. Of course, other sensing circuits may be available depending on need and/or desire. In reference to arrhythmias, as used herein, "sensing" is reserved for the noting of an electrical signal or obtaining data (information), and "detection" is the processing (analysis) of these sensed signals and noting the presence of an arrhythmia or of a precursor or other factor that may indicate a risk of or likelihood of an imminent onset of an arrhythmia.

Such an exemplary detection module 234, optionally uses timing intervals between sensed events (e.g., P-waves, R-waves, and depolarization signals associated with fibrillation which are sometimes referred to as "F-waves" or "Fib-waves") and to perform one or more comparisons to a predefined rate zone limit (i.e., bradycardia, normal, low rate VT, high rate VT, and fibrillation rate zones) and/or various other characteristics (e.g., sudden onset, stability, physiologic sensors, and morphology, etc.) in order to determine the type of remedial therapy (e.g., anti-arrhythmia, etc.) that is desired or needed (e.g., bradycardia pacing, anti-tachycardia pacing, cardioversion shocks or defibrillation shocks, collectively referred to as "tiered therapy"). Similar rules can be applied to the atrial channel to determine if there is an atrial tachyarrhythmia or atrial fibrillation with appropriate classification and intervention. Such a module is optionally suitable for performing various exemplary methods described herein. For example, such a module optionally allows for analyses related to action potentials (e.g., MAPs, T waves, etc.) and characteristics thereof (e.g., alternans, activation times, repolarization times, derivatives, etc.).

Cardiac signals are also applied to inputs of an analog-to-digital (A/D) data acquisition system 252. The data acquisition system 252 is configured to acquire intracardiac electrogram signals, convert the raw analog data into a digital signal, and store the digital signals for later processing and/or telemetric transmission to an external device 254. The data acquisition system 252 is coupled to the right atrial lead 104, the coronary sinus lead 106, the right ventricular lead 108 and/or the nerve stimulation lead through the switch 226 to sample cardiac signals across any pair of desired electrodes.

The microcontroller 220 is further coupled to a memory 260 by a suitable data/address bus 262, wherein the programmable operating parameters used by the microcontroller 220 are stored and modified, as required, in order to customize the operation of the stimulation device 100 to suit the needs of a particular patient. Such operating parameters define, for example, pacing pulse amplitude, pulse duration, electrode polarity, rate, sensitivity, automatic features, arrhythmia detection criteria, and the amplitude, waveshape, number of pulses, and vector of each shocking pulse to be delivered to the patient's heart 102 within each respective tier of therapy. One feature of the described embodiments is the ability to sense and store a relatively large amount of data (e.g., from the data acquisition system 252), which data may then be used for subsequent analysis to guide the programming of the device.

Advantageously, the operating parameters of the implantable device 100 may be non-invasively programmed into the memory 260 through a telemetry circuit 264 in telemetric communication via communication link 266 with the external device 254, such as a programmer, transtelephonic transceiver, or a diagnostic system analyzer. The microcontroller 220 activates the telemetry circuit 264 with a control signal 268. The telemetry circuit 264 advantageously allows intracardiac electrograms and status information relating to the operation of the device 100 (as contained in the microcontroller 220 or memory 260) to be sent to the external device 254 through an established communication link 266.

The stimulation device 100 can further include a physiologic sensor 270, commonly referred to as a "rate-responsive" sensor because it is typically used to adjust pacing stimulation rate according to the exercise state of the patient. However, the physiological sensor 270 may further be used to detect changes in cardiac output (see, e.g., U.S. Pat. No. 6,314,323, which discusses a pressure sensor adapted to sense pressure in a right ventricle and to generate an electrical pressure signal corresponding to the sensed pressure, an integrator supplied with the pressure signal which integrates the pressure signal between a start time and a stop time to produce an integration result that corresponds to cardiac output), changes in the physiological condition of the heart, or diurnal changes in activity (e.g., detecting sleep and wake states). Accordingly, the microcontroller 220 responds by adjusting the various pacing parameters (such as rate, AV Delay, V-V Delay, etc.) at which the atrial and ventricular pulse generators, 222 and 224, generate stimulation pulses.

While shown as being included within the stimulation device 100, it is to be understood that the physiologic sensor 270 may also be external to the stimulation device 100, yet still be implanted within or carried by the patient. Examples of physiologic sensors that may be implemented in device 100 include known sensors that, for example, sense respiration rate, pH of blood, ventricular gradient, cardiac output, preload, afterload, contractility, and so forth. Another sensor that may be used is one that detects activity variance, wherein an activity sensor is monitored diurnally to detect the low variance in the measurement corresponding to the sleep state. For a complete description of the activity variance sensor, the reader is directed to U.S. Patent No. 5,476,483 (Bornzin et al.).

More specifically, the physiological sensors 270 optionally include sensors for detecting movement and minute ventilation in the patient. The physiological sensors 270 may include a position sensor and/or a minute ventilation (MV) sensor to sense minute ventilation, which is defined as the total volume of air that moves in and out of a patient's lungs in a minute. Signals generated by the position sensor and MV sensor are passed to the microcontroller 220 for analysis in determining whether to adjust the pacing rate, etc. The microcontroller 220 monitors the signals for indications of the patient's position and activity status, such as whether the patient is climbing upstairs or descending downstairs or whether the patient is sitting up after lying down.

The stimulation device additionally includes a battery 276 that provides operating power to all of the circuits shown in Fig. 2. For the stimulation device 100, which employs shocking therapy, the battery 276 is capable of operating at low current drains for long periods of time (e.g., preferably less than 10 □A), and is capable of providing high-current pulses (for capacitor charging) when the patient requires a shock pulse (e.g., preferably, in excess of 2 A, at voltages above 2 V, for periods of 10 seconds or more). The battery 276 also desirably has a predictable discharge characteristic so that elective replacement time can be detected.

The stimulation device 100 can further include magnet detection circuitry (not shown), coupled to the microcontroller 220, to detect when a magnet is placed over the stimulation device 100. A magnet may be used by a clinician to perform various test functions of the stimulation device 100 and/or to signal the microcontroller 220 that the external programmer 254 is in place to receive or transmit data to the microcontroller 220 through the telemetry circuits 264.

The stimulation device 100 further includes an impedance measuring circuit 278 that is enabled by the microcontroller 220 via a control signal 280. The known uses for an impedance measuring circuit 278 include, but are not limited to, lead impedance surveillance during the acute and chronic phases for proper lead positioning or dislodgement; detecting operable electrodes and automatically switching to an operable pair if dislodgement occurs; measuring respiration or minute ventilation; measuring thoracic impedance for determining shock thresholds; detecting when the device has been implanted; measuring stroke volume; and detecting the opening of heart valves, etc. The impedance measuring circuit 278 is advantageously coupled to the switch 226 so that any desired electrode may be used.

In the case where the stimulation device 100 is intended to operate as an implantable cardioverter/defibrillator (ICD) device, it detects the occurrence of an arrhythmia, and automatically applies an appropriate therapy to the heart aimed at terminating the detected arrhythmia. To this end, the microcontroller 220 further controls a shocking circuit 282 by way of a control signal 284. The shocking circuit 282 generates shocking pulses of low (e.g., up to 0.5 J), moderate (e.g., 0.5 J to 10 J), or high energy (e.g., 11 J to 40 J), as controlled by the microcontroller 220. Such shocking pulses are applied to the patient's heart 102 through at least two shocking electrodes, and as shown in this embodiment, selected from the left atrial coil electrode 126, the RV coil electrode 132, and/or the SVC coil electrode 134. As noted above, the housing 200 may act as an active electrode in combination with the RV electrode 132, or as part of a split electrical vector using the SVC coil electrode 134 or the left atrial coil electrode 126 (i.e., using the RV electrode as a common electrode).

Cardioversion level shocks are generally considered to be of low to moderate energy level (so as to minimize pain felt by the patient), and/or synchronized with an R-wave and/or pertaining to the treatment of tachycardia. Defibrillation shocks are generally of moderate to high energy level (e.g., corresponding to thresholds in the range of approximately 5 J to 40 J), delivered asynchronously (since R-waves may be too disorganized), and pertaining exclusively to the treatment of fibrillation. Accordingly, the microcontroller 220 is capable of controlling the synchronous or asynchronous delivery of the shocking pulses.

### Phases of Patient and/or Device Care

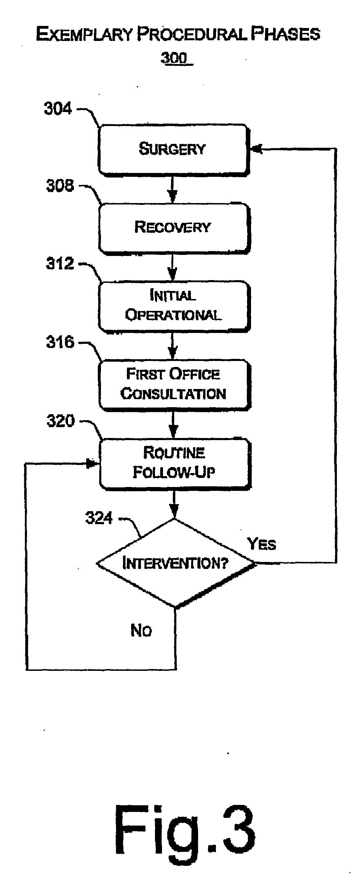

Fig. 3 shows various phases of patient and/or device care 300. While clinical procedures often commence prior to surgery related to implantation of a device, in this example, exemplary phases commence with surgery 304. After implantation surgery, a patient enters a recovery phase 308 wherein a patient is taken to a recovery room or a hospital room, typically for a stay from several hours to several days. During this phase, a care provider will generally monitor the patient for symptoms of infection at or near the site of implantation. In the few weeks following surgery, a care provider will generally advise the patient to avoid sudden, jerky movements with her arms, or stretching or reaching over her head. A care provider will generally let the patient know which activities can be resumed and when.

Benefits derived from the implanted device can be expected to appear within the weeks following implantation. In this initial operational phase 312, care providers generally urge patients to communicate any new sensations experienced as a result of the device implantation. Such information may later help the care provider to adjust programmable settings of the device to suit a patient's particular needs.

Adjustments may occur during a first post-implant office consultation 316 with a care provider specialized in operation of the implanted device, usually within a few weeks of surgery. This consultation allows the care provider to determine how well the device is working and to make minor adjustments, if required.

A routine follow-up phase 320 continues thereafter. During follow-up visits, a care provider may use a transmitter and/or receiver unit to communicate with the implanted device. The transmitter and/or receiver unit usually includes a wand connected to a computer. This unit can allow a care provider to adjust and/or fine-tune various settings. In a typical procedure, a care provider places the wand over the implanted device to allow for communication with the implanted device. This procedure typically takes a few minutes and can be performed during a routine office consultation.

Routine consultations, whether at a distance or in-person, may indicate that intervention is desirable or necessary. A decision block 324 represents decision making procedures related to whether intervention is required. In this example, if surgical intervention is required, then a patient may repeat the surgery phase 304. However, if no surgical intervention is required, then routine follow-up 320 will continue.

At some point in time, surgical intervention may be required to replace a power source of the implanted device, to service the implanted device, to service a lead, etc. At present, most implanted devices require service via surgical intervention in a time frame from approximately 5 to approximately 10 years or more, with an average of about seven years. Longevity depends upon power demand and/or other factors. Power demand is typically a function of how much energy is required to pace a heart and how an implanted device is programmed for operation. Assuming a minimum of four distance or office consultations a year, a patient may expect a minimum of approximately 20 to approximately 40 consultations prior to a need for surgical intervention. Of course, patient morbidity and/or other factors may have a direct impact on the minimum number of consultations.

Patients having an implanted device are often assigned and given a card, a bracelet or other identifier that allows other to readily know that a patient has an implanted device. Patients often get a temporary identifier at the time of implant; while a more permanent identifier is typically provided at a later date by the device manufacturer. At present, such an identifier includes information on the type of device and other information set by the manufacture, for example, based on an initial operational program. The identifier aids in the case of a medical emergency and patients are urged to keep it with them at all times.

### Exemplary Follow-up Guidelines

Various countries have instituted follow-up guidelines for patients having implanted cardiac therapy devices. For example, Fraser et al., "Guidelines for pacemaker follow-up in Canada: A consensus statement of the Canadian Working Group on Cardiac Pacing", Canadian Journal of Cardiology, 16(3): 355-363J (2000), reported that a need exists for follow-up guidelines. Fraser et al. (or the "Canadian report"), also noted that physicians played a primary role in establishing policies and protocols and in approving programming changes while associated professionals conducted follow-up assessment and made programming changes in absence of direct physician supervision. With respect to the various phases, Fraser, et al., recommended a post-implantation phase within 72 hours of implantation (e.g., the phase 312), a two week to twelve week phase (e.g., the phase 316), a six month phase and an ongoing annual phase thereafter (e.g., the phase 320).

In the United States, the American College of Cardiology and the American Heart Association set forth guidelines in a report by Gregoratos, et al., entitled "ACC/AHA guidelines for implantation of cardiac pacemakers and antiarrhythmia devices: a report of the American College of Cardiology/American Heart Association Task Force on Practice Guidelines (Committee on Pacemaker Implantation)", J. Am. Coll. Cardiol., 31(5):1175-1209 (1998), referred to herein as the "ACC/AHA report". The ACC/AHA report recommended that follow-up be individualized in accordance with the patient's clinical status. The ACC/AHA report recommends a predischarge and/or subsequent postoperative electrophysiological testing phase (e.g., the phase 312 or the phase 316). Thereafter, the report states that implanted devices should be followed at intervals of approximately 1 month to approximately 4 months, depending on the device model and the patient's clinical status (e.g., the phase 320). The ACC/AHA report notes that manufacturer guidelines for device follow-up may vary with individual models. Further, distance (e.g., transtelephonic, etc.) follow-up should be supplemented by clinic visits at a minimum of 3 month intervals for patient and implanted device evaluation.

The ACC/AHA report notes that it is often necessary to reprogram the initially selected parameters either in the outpatient clinic or by electrophysiological testing. Further, when device function or concomitant antiarrhythmic therapy is modified, electrophysiological testing can be and often is required to evaluate sensing, pacing, or defibrillation functions of the device. The ACC/AHA report states that particular attention should be given to review of sensing parameters, programmed defibrillation and pacing therapies, device activation, and event logs; whereas, technical elements requiring review include battery status, lead system parameters, and elective replacement indicators.

The ACC/AHA report further notes that intervening evaluation of device function is often necessary, wherein patients experiencing device activation, with or without therapy, delivery should be evaluated shortly after the event until a regular acceptable pattern of patient symptomatology and tolerance for such events is established and device behavior is deemed reliable, safe, and effective. Yet further, like the Canadian report, the ACC/AHA recommends that recipients be encouraged to carry proper identification and information about their device at all times. Care providers should also be cognizant of patients experiencing transient or sustained emotional disturbances and the fact that education and psychological support before, during, and after device insertion are highly desirable and can improve the patient's quality of life.

### Exemplary Patient Characteristics Germane to Follow-up Consultations

Any phase of follow-up should consider or account for various patient-related characteristics. For example, a care provider may consider patient dependency on pacing, stability of rhythm and cardiovascular symptoms, high or unstable pacing thresholds or low and stable pacing thresholds, a patient's ability to report symptoms accurately, a patient's distance from a follow-up clinic, general health or lack thereof. Of course, implanted device characteristics may be considered as well.

### Exemplary Early Post-Op Consultations (e.g., the phase 308 and/or the phase 312)

According to the Canadian report, an early post-implantation or post-operative consultation should aim to assess the patient-implanted device interface; determine normal implanted device function; determine appropriate implanted device programs; set up appropriate diagnostics; document initial telemetry values; ensure the absence of operative complications; educate and emotionally support the patient and family; provide the patient with implanted device identification that includes the model number and manufacturer of all implanted hardware.

At this early consultation, appropriate function (e.g., pacing, sensing, etc.) should be confirmed by review of telemetry strips postoperatively, ECG, etc. For example, a multi-electrode ECG may be included in a patient's record as a reference. The care provider should also assess pacing and sensing thresholds where appropriate along with any reprogramming to adjust implanted device operations (e.g., pacing rates, atrio-ventricular delay and/or other parameters). Further, where appropriate, diagnostic features of an implanted device should be adjusted to meet specific patient needs and answer specific clinical questions (e.g., rate ranges, counts of premature ventricular complexes, rate drop, etc.).

Towards the later end of this period of consultation, a care provider should measure, where appropriate, stimulation thresholds and programming of output parameters to ensure reliable capture and optimization of device longevity; measure sensing thresholds and determination of the implanted device response to strenuous arm motion (e.g., myopotential, etc.), if appropriate; review diagnostics and fine tuning of therapy (e.g., sensor rate, adaptive atrio-ventricular delay, mode switching, auto threshold, etc.); carefully assess the wound; establish whether the goals of the implanted device therapy have been or are being achieved; and address patient and family concerns.

### Exemplary Early Office Consultation (e.g., the phase 316)

As mentioned, an early office consultation phase (e.g., the phase 316) may follow the post-operative phases (e.g., the phases 308, 312). During this phase, a care provider should ensure normal function, sensing and capture thresholds, etc.; assess symptoms; provide a patient with education and support; consider programming that can maximize longevity (if not done at an earlier visit); optimize implanted device function; review diagnostic data; establish communication with a primary care physician or other primary care provider. During such visits, the Canadian report recommends that a targeted cardiovascular history be taken and a limited cardiovascular examination.

During all routine follow-up consultations, real-time free-running rhythm strips may be required to document functions such as sensing, capture and rhythm. Stability of underlying rhythm should be assessed and documented. Rhythm strips can be obtained from the programmer and may be associated with telemetered 'marker channels' and intracardiac electrograms (IEGMs). The implanted device may be interrogated, and the communicated data (e.g., telemetry data) may be reviewed and compared with baseline post-operative phase data (e.g., from the phase 308 and/or the phase 312). Often, upon review, diagnostic data should be reviewed and used to adjust appropriate implanted device features, if necessary (e.g., atrio-ventricular delay, rate/sensor response parameters, mode switching, etc.).

A care provider should be able to use available diagnostics and understand such diagnostics for each type of implanted device under examination. Such an understanding and awareness can assist greatly in patient care and management. Further, a sound understanding of features associated with any particular implanted device can assist greatly in patient care and management. For example, where appropriate, an understanding of an implanted device's capture and sensing thresholds, pulse amplitude, pulse duration, sensitivity, etc. can improve patient quality of life and/or patient longevity.

### Exemplary On-going in Office Consultations (e.g., the phase 320)

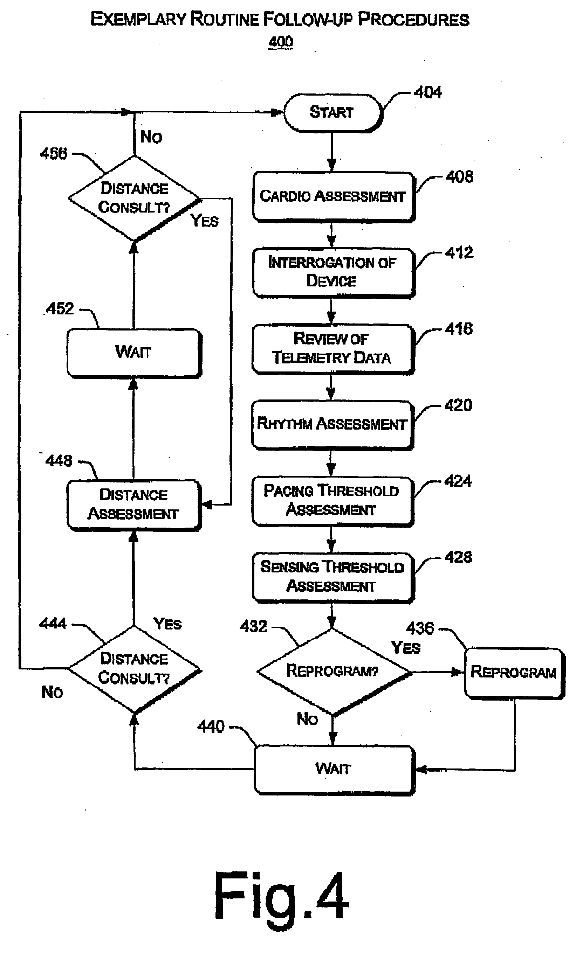

The Canadian report recommends various assessments during a typical follow-up consultation. Fig. 4 shows a block diagram of various exemplary assessments or procedures 400 that may be performed during an in office consultation. The exemplary procedures 400 commence in a start block 404. Next, in a cardio assessment block 408, a care provider performs a cardiovascular assessment. In general, a cardiovascular assessment abides by certain procedures. Such procedures may be patient specific, clinic specific, implanted device specific and/or care provider specific. Further, such procedures may change based on patient needs, clinic policy, regulatory policy, insurer policy, manufacturer policy, and/or care provider opinion.

An exemplary cardiovascular assessment 408 may include assessment of a patient's past and present condition. For example, such an assessment may include a questionnaire regarding patient history of dizziness, syncope, palpitations, dyspnea, fatigue, chest pain, medication, etc. As for present condition, such an assessment may include observation or measurement of blood pressure, heart rate and rhythm (e.g., electrocardiographic rhythm with implanted device "off" and "on"), respiratory rate and effort, heart sounds, breath sounds, edema, skin color, wound and site assessment, neck veins for cannon waves, etc. Each of these observations or measurements may require adherence to particular procedures. Or, such observations or measurements may follow a particular care provider's personal procedures (e.g., as taught, trained, personal choice, etc.).

An interrogation of an implanted device 412 assessment follows the cardiovascular assessment 408 in the exemplary routine follow-up procedures 400. At this point, a care provider may perform procedures to create or recall a history of the implanted device. For example, a history may include age of a lead and/or a pulse generator, specific operating characteristics, previous clinical complications, previous hardware complications (e.g., advisory hardware, lead fracture, abandoned leads, etc.), known reliability of implanted hardware, etc.

According to the exemplary procedures 400, an assessment or review of communicated data (e.g., telemetry data) 416 follows the interrogation 412. A care provider may perform procedures that determine available communication features of an implanted device, which typically vary with respect to implanted device manufacturer and model. The assessment 416 may include communication of data specific to an implanted device and/or communication of data specific to a patient and/or to interaction between a patient and an implanted device. During the data assessment 416, a care provider may perform procedures that determine programmed settings including last programmed date, date stamping of changes in battery status, model number, patient information and implantation date, battery status (e.g., cell impedance, voltage, energy, charge, current drain, etc.), lead information (type of lead, lead impedance, polarity, etc.), etc. During the data assessment 416, a care provider may follow procedures related to diagnostic data collected by an implanted device. For example, an implanted device may collect heart rate histograms that optionally use user-defined high rate counters, count ventricular arrhythmias and/or atrial tachycardia, sensor rate histograms, intracardiac electrograms (IEGMs), telemetered markers, diagnostics specific to therapies such as heart rate drop and mode switching, lead impedance trends with respect to time, capture and sensing thresholds with respect to time, indicators as to percentage of pacing and sensing in each chamber, automatic features such as automatic rate sensor changes, pacing thresholds and changes in device output.

Once a care provider has collected information, then, in a rhythm assessment block 420, a care provider should perform various procedures that use the information to assess rhythm. Of course, a care provider may interact with an implanted device at this time as well to ascertain information related to rhythm. A rhythm assessment may include procedures for assessing atrioventricular conduction and ventriculoatrial conduction.

The exemplary routine follow-up procedures 400 may also include various threshold assessments, where appropriate. For example, a pacing threshold assessment block 424 (e.g., to assess capture, etc.) and a sensing threshold assessment block 428 may include procedures specific to an implanted device, a patient, a care provider, etc.

The exemplary routine follow-up procedures 400 also include a decision block 432 for deciding whether reprogramming is required or desired. If the decision block 432, for example, based on a care provider's knowledge or manufacturer's recommendation, decides that a patient's implanted device should be reprogrammed, then a reprogram block 436 follows; otherwise, a wait block 440 follows, wherein a wait period occurs. After the reprogram block 436, a wait block may also be instituted (e.g., the wait block 436).

Thereafter, in another decision block 444, a decision occurs with respect to distance consultation or assessment. If a decision is made to forgo distance consultation, then a patient may not be examined until the start of another in office consultation (e.g., at the start block 404). However, if a decision is made to perform one or more distance assessments prior to a subsequent in office consultation, then a distance assessment block 448 occurs, which includes various procedures, which may be at least to some degree automated. After the distance assessment block 448, another wait block 452 institutes an appropriate wait period. After the wait period, assuming no adverse symptoms requiring immediate attention, another decision block 456 may decide whether multiple distance assessments should be performed prior to a subsequent in office consultation. If another distance assessment is desired or required, then, in the distance assessment block 448, another distance assessment may be performed. Otherwise, the patient continues, at an appropriate time, with a subsequent in office consultation.

With respect to distance assessments (e.g., the block 448), such assessments may include monitoring function of an implanted device in an infirm or geographically isolated patient; monitoring for an indication of elective replacement; monitoring for intermittent sustained symptoms or arrhythmia; monitoring implanted devices on recall where failure may be characterised by a permanent change in function, such as, rate, mode or sensing parameter, which can be detected by, for example, a rhythm strip acquired at a distance.

The Canadian report suggests that a typical follow-up visit include a targeted cardiovascular assessment, interrogation of the pacing system, review of telemetered data, assessment of the underlying rhythm, assessment of pacing and sensing thresholds, and appropriate reprogramming of pacing parameters to optimize device function and longevity. According to various exemplary methods described herein, such recommended procedures serve to illustrate benefits and/or operation of the various exemplary methods.

The Canadian report also recommends the following information to be kept in a patient record: demographic information on patient (name, age, telephone number or other means of contract, next of kin or alternative way of contacting a patient); responsible physician or care provider; pacemaker operative record; manufacturer, model number and serial number of all implanted hardware; records of data from each follow-up visit including programmed setting, telemetry threshold evaluations and rhythm strips; patient symptoms or complaints; evaluation of the pacemaker site; documentation of hardware advisory or recall, or any surgical complications; and current medications. Of course, other information may also be kept in a patient record; however, a decision should be made as to the value or usefulness of the information to prevent extraneous information from being included.

### Current Status of Follow-up Consultations

The nature of follow-up consultations for patients having implanted devices varies. As already mentioned, distance consultations (e.g., telemetry, etc.) typically occur more frequently than in office consultations. The ACC/AHA guidelines recommend distance consultations as frequent as once per month and in office consultations at a minimum of once every three months. Thus, a patient may expect 12 distance consultations per year and four in office consultations per year. Over the life of an implanted device, assuming a five to ten year implanted device life-span at these aforementioned frequencies, a patient may expect from sixty to one-hundred distance consultations and from twenty to forty in office consultations.

With respect to time of an in office consultation, according to the Canadian report, on average, a typical follow-up consultation lasted about 18 minutes for a patient with a single chamber device and about 29 minutes for a patient with a dual chamber device; while a physical assessment component of a consultation typically took about 6.5 minutes. Thus, for purposes of illustration, an in office consultation, with a patient present, may take about one-half hour of care provider time. With respect to consultation frequency and total number of consultations, a patient may expect approximately 10 hours to approximately 20 hours of in office consultation time with a care provider over the life-span of an implanted device.

While the actual hours may not appear to be extraordinary, when one considers that a major clinic may perform over 500 implant device surgeries per year, one year of patient load may translate into approximately 5,000 hours to approximately 10,000 hours of in office consultation time over a period of approximately five to ten years. When this is multiplied by several years at such a per year patient load, the scheduling and resource demand on a clinic may be substantial. Thus, a reduction consultation time alone can translate into a substantial reduction in office consultation hours. For example, a five minute time reduction (e.g., about a 17% reduction in time) for an in office consultation may translate into a reduction of about 850 hours to about 1700 hours for one year of patient load over the life-span of an implanted device.

### Exemplary Procedure Tracking and/or Analysis Methods

The example with respect to time touches on but one aspect of the current status of follow-up visits. As described herein, exemplary methods and/or devices allow for procedure tracking and/or analysis of procedures in a manner that can help to reduce consultation time, harmonize consultation procedures, reduce insurance costs, improve training, design better implanted devices, etc. For example, various exemplary methods and/or devices may assist and reduce the time required for care providers or other clinicians to learn aspects or features of various implanted devices; propose one or more custom automated follow-up protocols for each care provider; assist and reduce the time required for a follow-up consultation (e.g., session); allow for an on line review of historical diagnostic data and programming changes made to an implanted device when necessary; assist in the diagnosis of patient symptoms; recommend appropriate programming changes necessary for a particular patient or group of patients.

Reduces the time and effort required by clinicians to learn a new implantable medical device; reduces follow-up time; ability to diagnose patients based on sound and proven diagnosis and treatments; ability to get references from the system as what has been done to patients with similar symptoms; ability to review historical data real time based when necessary.

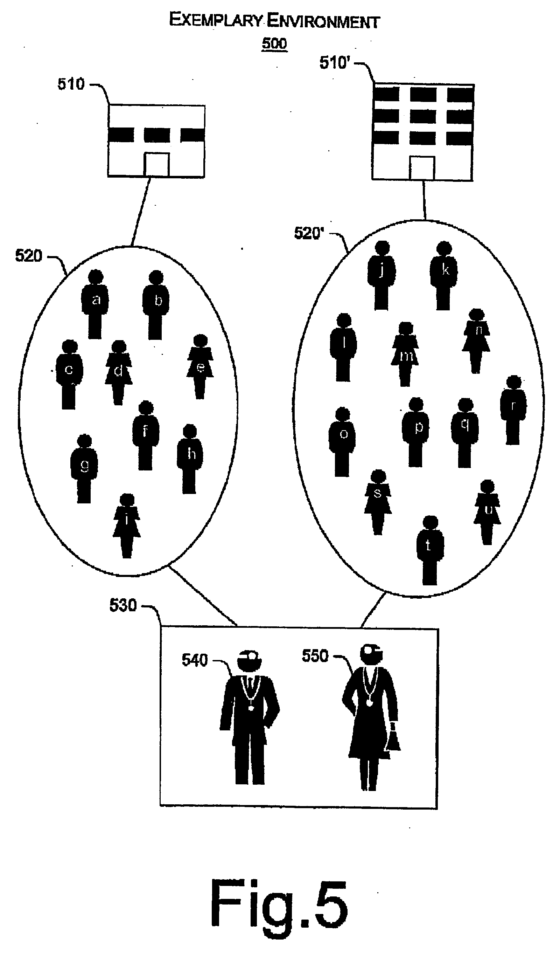

Fig. 5 shows an exemplary environment 500 that includes implantable device manufacturers 510, 510', patient populations 520, 520' and a clinic 530 having care providers 532, 534. The patient population 520 includes patients labeled "a" through "i", while the patient population 520' includes patients labeled "j" through "u". Various exemplary methods described herein pertain to care providers and their relationship to patients in patient populations. In the exemplary environment 500, the care providers 540, 550 provide care for various patients within the patient populations 520, 520'. In the exemplary environment 500, the patient populations 520, 520' are defined with respect to the manufacturers 510, 510' of their respective implanted devices. Of course, patient populations may be defined with respect to other characteristics such as age, sex, disease, etc. In this example, defining patient populations with respect to manufacturer allows for tracking and/or analysis of procedures that extend from manufacturer to patient to care provider. For example, limitations of various assessments are often dictated by bounds established by capabilities of an implanted device, which, in turn, are dictated at least in part by an implantable device manufacturer.

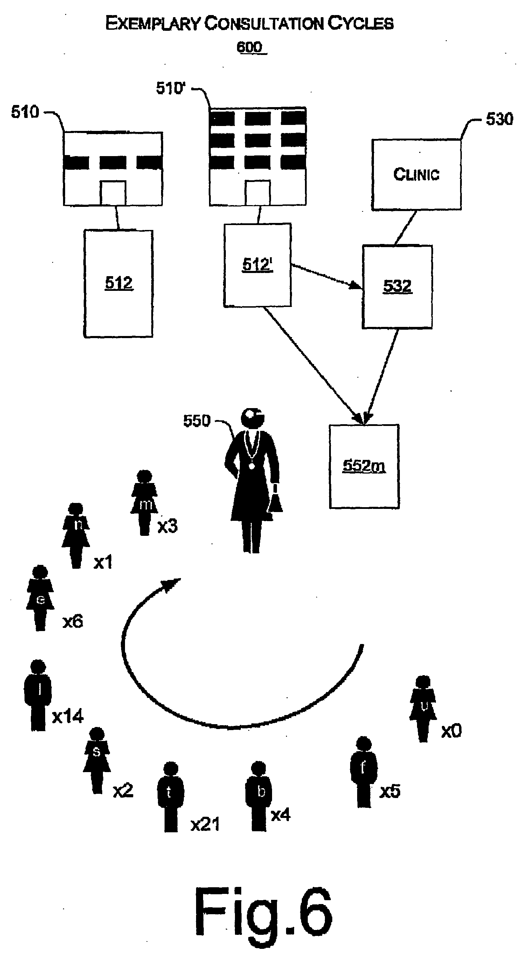

Fig. 6 shows an exemplary cycle of patient consultations 600 for various patients of the patient populations 520, 520' and the care provider 550 of Fig. 5. In this example, the care provider 550 provides care for patient "m", which has an implanted device from the manufacturer 510'. The manufacturer 510' has associated procedures or a protocol 512' that are suitable for the implanted device of the patient "m". Further, the clinic 530 has procedures or a protocol 532 for patients such as the patient "m". During the consultation, the care provider 550 provides care for the patient "m" according to procedures or a protocol 552m, which accounts for the manufacturer's recommendations and those of the clinic 530. The protocol 552m may optionally account for the care provider's 550 personal preferences or training.

In general, the procedures or protocol suitable for patient "m" (e.g., the procedures or the protocol 552m), are performed according to the care provider's 550 memory, the clinic's 530 records, which may include some data or may simply be a set of procedures that do not necessarily depend on characteristics related a particular patient or a particular patient's history. For example, such a set of procedures may simply pertain to the type of implanted device (e.g., pacemaker, ICD, etc.). Such procedures may be updated from time-to-time based on information from a manufacturer.

In the example 600, every patient has a corresponding number of visits. For example, patient "m" has been seen by the care provider 550 on three occasions for in office consultation related to the patient's implanted device; whereas, patient "t" has been seen by the care provider 550 on twenty-one occasions for in office consultation related to the patient's implanted device. Thus, the care provider 550 may have a better memory of procedures that pertain to patient "t" in comparison to procedures for patient "m". According to these exemplary consultation cycles 600, if the care provider 550 is not available for a consultation of patient "t", then the fact that patient "t" has been seen at the clinic twenty-one times may provide little benefit to, for example, a consultation performed by the care provider 540. Hence, as described below, an exemplary method tracks and/or analyzes procedures optionally on a patient-by-patient basis for individual care providers. Such an exemplary method may capture the procedural history of prior patient consultations and optionally analyze such information in an effort to improve patient care and/or ensure a certain level of patient care.

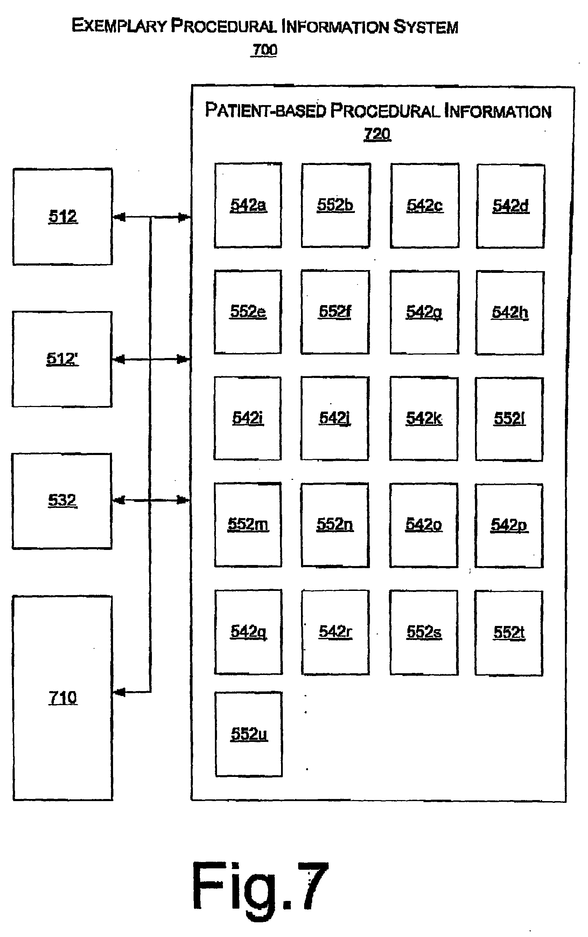

Fig. 7 shows an exemplary procedural information system 700. The exemplary system includes a computer 710 (e.g., a server, etc.) for collecting, tracking and/or analyzing patient-based procedural information 720 and optionally other procedural information, such as, but not limited to, manufacturer information 512, 512' and clinic information 532. In this example, the patient-based procedural information 720 includes individual procedural records for each patient (e.g., the patients "a" through "u") wherein the records also pertain to a care provider (e.g., the care provider 540 or the care provider 550). Hence, individual patient records are labeled either 542 (e.g., for the care provider 540) or 552 (e.g., for the care provider 550). Of course, an individual record may pertain to more than one care provider. In such instances, individual patient records are optionally assigned to a primary care provider.

The computer 710 allows for updating of individual patient records, for example, based on subsequent consultations, clinic information, manufacturer information and/or other information that may impact a procedure (e.g., improve a procedure, etc.). The computer 710 may also include software, such as, database software, statistical analysis software, input software, output software, etc. For example, statistical analysis software may be used to perform statistical analyses on an intra-record basis and/or on an inter-record basis.

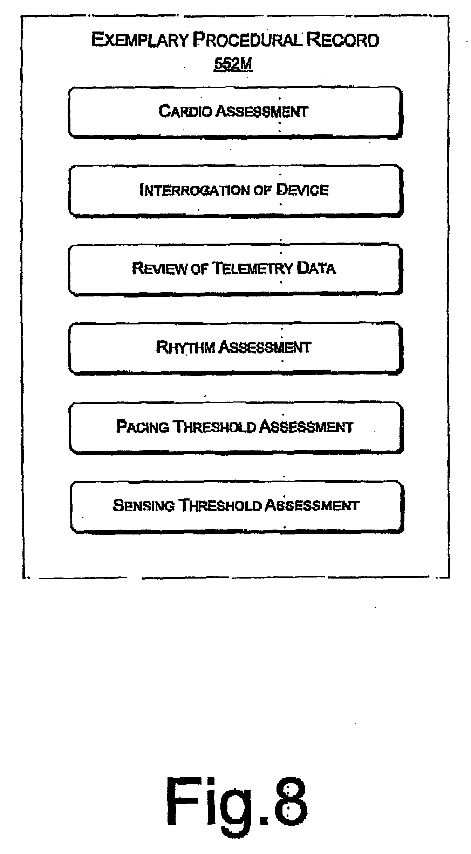

Fig. 8 shows an exemplary patient record 552m that includes procedural information for various procedures described with reference to Fig. 4. For example, the exemplary patient record 552m includes procedural information for a cardiovascular assessment, an interrogation of an implanted device, a review of telemetry data, a rhythm assessment, a pacing threshold assessment and a sensing threshold assessment. Where appropriate, each patient has an associated patient record that includes procedural information and, in particular, procedural information pertaining to procedures performed by a care provider.

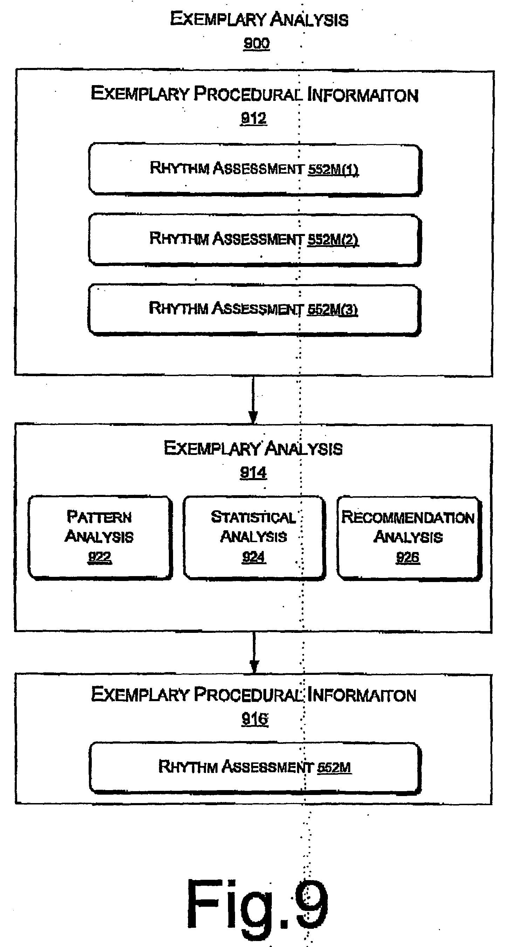

Fig. 9 shows an exemplary analysis of procedural information 900. In this example, the patient "m" has seen the care provider 550 on three occasions; hence, for example, three procedural histories 552m(1), 552m(2) and 552m(3) form exemplary procedural information 912. On each of these occasions, the care provider 550 performed a rhythm assessment according to a protocol or one or more procedures. For example, the care provider 550 may determine stability of the underlying rhythm through a review of rhythm strips obtained from the implanted device during the in office consultation and/or from prior distance consultations. Such a rhythm assessment may include use of telemetered marker channels, intracardiac electrograms and/or previously acquired baseline or other information. Hence, a procedure or protocol for rhythm assessment may include the following:
1. Enter telemetry data from inter-office consultation distance consultations into a diagnostic program;
2. Enter telemetry data from present office consultation into the diagnostic program;
3. Retrieve and enter other historical telemetry data into the diagnostic program;
4. Retrieve and enter baseline data into the diagnostic program;
5. Use the diagnostic program to analyze the telemetry data;
6. Use the diagnostic program to compare the analyzed data with baseline data; and
7. Determine prognosis and action steps.

Of course, some of these procedures may not be needed for a preliminary or a first in office consultation. Thus, the procedural history for the rhythm assessment may vary. For example, the first rhythm assessment procedures 522m(1) may differ from the second rhythm assessment procedures 522m(2) and the third rhythm assessment procedures 552m(3).

The procedural information 912 is optionally collected in real-time during an in office consultation and/or entered after an in office consultation or a portion thereof. In one example, a computer and associated display guides a care provider through various procedures. In this example, the care provider may interact with the computer to select and/or affirm that certain procedures were followed and/or to note an order in which certain procedures were followed. Such a computer may operate via voice command, a keyboard, a touch screen or other input. Further, interaction may include data entry (e.g., manually or automatically) from a patient, a care provider and/or an implanted device (e.g., via a telemetry or other communication device). Hence, use of such a computer allows a care provider to record information such as procedural information and data germane to a patient and/or an implanted device.

Prior to a fourth rhythm assessment, an exemplary analysis 914 analyzes the procedural information 912. In this example, the exemplary analysis 914 includes a pattern analysis 922, a confidence analysis 924 and a recommendation analysis 926. The pattern analysis 922 analyzes the procedural information 912 for particular patterns. For example, the pattern analysis 922 optionally searches the procedural information 912 for particular types of repeated behavior patterns in previous performed procedures. Such a pattern search optionally accounts for procedures described in guidelines. For example, the pattern analysis 922 may rely on known patterns and recognition of such known patterns in recorded procedural information. The pattern analysis 922 optionally assigns a match value to any recognition. For example, if a known pattern includes three hypothetical steps performed within a time interval of 10 minutes and recorded procedural information includes two of the steps and another third step performed within a time interval of 9 minutes, the exemplary pattern analysis may assign a match of steps factor (e.g., 2 of 3 steps) and it may assign a match of time factor (e.g., - 1 minute or 10% time savings). Note that such assigned factors may indicate an opportunity to optimize procedures for an in office consultation. Of course, results and patient outcome may be considered prior to implementing or recommending any particular procedures.

In the example 900, the pattern analysis 922 may analyze the procedural information for the three rhythm assessment 552m(1), 552m(2) and 552m(3). After the pattern analysis 922, the exemplary analysis 914 may use a statistical analysis 924 that allows for various statistical techniques, such as, a confidence, mean, deviation, etc. The statistical analysis 924 may assign a confidence level or levels to the procedural information for the rhythm assessments 552m(1), 552m(2) and 552m(3). Such statistical techniques may help in recommending procedures, for example, via a recommendation analysis 926.

The recommendation analysis 926 optionally relies on the pattern analysis 922, the statistical analysis 924 and/or known guidelines. Yet further, the exemplary analysis 912 may use information for other patients, care providers, clinics, implanted devices, etc., to arrive at recommended procedures.

Regarding the patient "m", as cared for by care provider 550, the exemplary analysis 914 uses the exemplary procedural information 912 from three prior consultations performed by the care provider 550 to recommend procedural information 916 (e.g., one or more procedures) for a subsequent consultation with patient "m" (e.g., a fourth consultation). The exemplary procedural information 916 may be output as a hard copy and/or used in conjunction with a computerized system that operates automatically and/or interactively with a care provider. In this regard, the exemplary analysis 914 learns about the care provider 550 and recommends procedures that may allow the care provider 550, or another care provider, to provide better care and/or more efficient care for patient "m" and/or other patients.

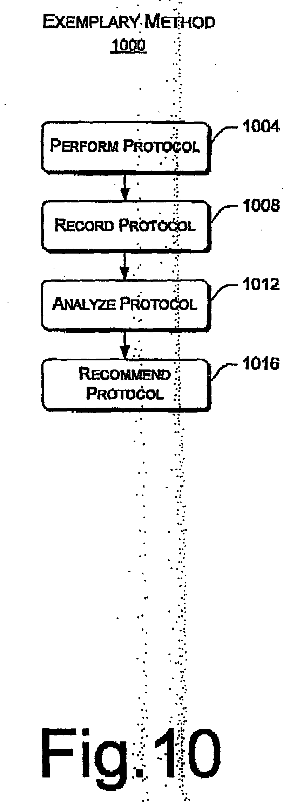

Fig. 10 shows an exemplary method 1000 for recommending procedures or protocols. In a performance block 1004, a care provider performs a follow-up protocol. Simultaneously, or thereafter, a record block 1008 records the follow-up protocol. An analysis block 1012 follows wherein an analysis of the follow-up protocol occurs, for example, with respect to previously performed or previously recommended protocols (e.g., pertaining to the same patient or other patient, the same care provider or other care provider, the same clinic or other clinic, the same implanted device manufacturer or other manufacturer, the same model implanted device or other implanted device, etc.). On the basis of the analysis, in a recommendation block 1016, the exemplary method 1000 recommends a protocol for future use.

With respect to the recording (e.g., the record block 1008), a care provider may perform the follow-up consultation in conjunction with a computer-based data logging system. For example, a care provider may perform procedures using a computer and/or a specialized computer that can communicate with an implanted device. As the care provider performs the procedures, such a computer or computers can log procedures, such as, but not limited to, sequences in which implanted device features are accessed, data collected and/or interrogated, patient queried, etc. Such procedures may pertain to one or more of the exemplary procedures 400 of Fig. 4. Procedural information may then be stored locally and/or remotely. Further, such information or analyzed information is optionally stored on a card or memory device to be carried by a patient (e.g., consider the aforementioned patient identification card, which may include a memory strip or other memory).

In one example, procedural information logging includes date, time, care provider identification, implanted device identification and implant device features accessed during a procedure and optionally the order in which such features are accessed. Further recording may log diagnostic data, patient data (e.g., patient identification information, personal information, etc.), implant device operational parameters, etc.

With respect to diagnostics, procedural information may include a diagnosis. In such an instance, a procedural record may include information associated with procedures taken to arrive at the diagnosis. Referring to the exemplary system 700 of Fig. 7, a search of procedural records may allow for analysis of procedures with respect to diagnosis of particular diseases or conditions. For example, a care provider may perform various procedures to determine whether a patient has a normal QT interval, wherein an abnormally long QT interval may indicate "long QT syndrome". Long QT syndrome may be associated with conduction defects that can predispose affected patients to arrhythmia such as "Torsade de Pointes", which can, in turn, lead to sudden loss of consciousness (syncope) and even sudden cardiac death. Long QT syndrome may be associated with a patient's genetic makeup or be acquired (e.g., induced via medication or other environmental conditions).

Given a plurality of patient procedural records, a care provider may analyze the records to determine a procedure that has proven successful at diagnosing long QT syndrome. Of course, such an analysis, or search, may provide more than one procedure, wherein the care provider may then select one or more of the procedures and perform the one or more selected procedures to improve certainty of a diagnosis. A care provider may, in addition to or alternatively, analyze a plurality of procedures to arrive at a custom or hybrid procedure. Procedures are optionally classified as being capable of determining whether long QT syndrome has a genetic or other underlying cause. Further, such procedures may help a care provider determine one or more parameters to improve patient health and/or mitigate adverse effects of such a disease or condition.

An exemplary analysis module (e.g., such as or similar to the module 914), is optionally used to determine a procedure or protocol for diagnosis of one or more diseases and/or conditions related to a patient's heart. Such an exemplary analysis module may also determine one or more parameters to improve operation of an implanted device for a patient having or at risk of having the one or more diseases and/or conditions. This later determination may rely, at least in part, on another analysis of procedural information. For example, a first analysis may determine diagnostic procedures while a subsequent analysis may determine procedure and/or operation parameters aimed at treatment (e.g., including treatment aimed at quality of life, length of life, etc.).

### Exemplary Programming System

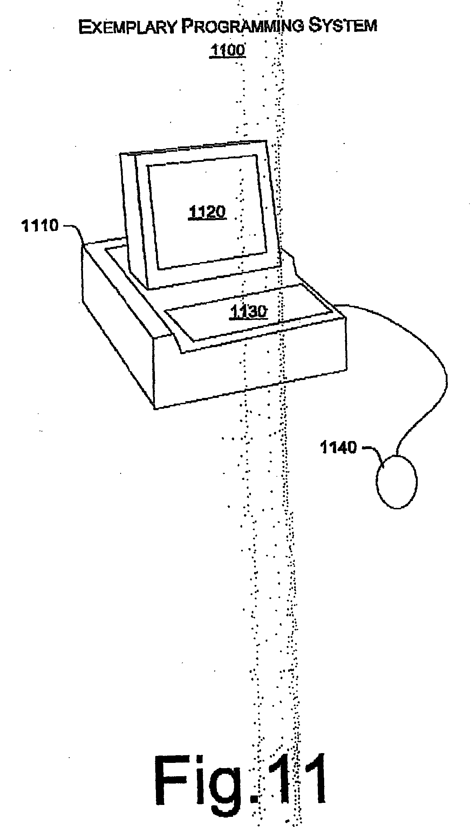

Fig. 11 shows an exemplary programming system 1100 for communicating with an implanted device (e.g., the device 100 of Figs. 1 and 2). The exemplary programming system 1100 includes a computer 1110, a display 1120, a keyboard 1130 and a telemetry probe 1140. The telemetry probe 1140 allows for communication with an implanted device. In general, such a probe is placed in proximity to an implanted device and communication is established via radio frequency transmission.

The term "programmer" or "programming system" is commonly used in the implanted device industry to describe a computing system that includes hardware and software that can operate to communicate with an implanted device. For example, St. Jude Medical, Inc. (St. Paul, MN) markets the Model 3510 programming system that includes a computer system-based programmer. Thus, in one example, the exemplary programming system 1100 includes various features of the Model 3510 programming system. The Model 3510 programming system includes a custom, portable computer system that features a large, color display on a touch-sensitive, active matrix LCD screen. The Model 3510 programmer can provide continuous, simultaneous display of surface ECGs, intracardiac electrograms, annotated event markers, and Electronic Calipers™, where an A paced and V sensed interval (e.g., AR interval) is automatically measured and reported, for example, on the programmer screen. The Model 3510 includes a full-size keyboard that allows for easy input of patient and other information. An automated follow-up feature guides this process and generates custom reports quickly and easily. The programmer then prints reports on a full-size sheet of paper for easy insertion into patient charts. The Model 3510 programming system is lightweight for easy portability. In addition, it can interface various commercially available data management systems.

More specific features of the Model 3510 programming system include a capability to collect up to 60 episodes and 25 minutes of continuous fully annotated stored electrograms with up to 32 seconds of pre-trigger information; a user-selectable standard automated follow-up protocol and up to 24 customizable automatic follow-up protocols; archive data storage that offers the ability to view and print information acquired during previous follow-up sessions; detection, therapy, and episode summaries; lifetime diagnostics, including pacing and charging history and event, heart rate, and sensor histograms.

As described herein, the exemplary programming system 1100 includes an ability to analyze follow-up procedures or protocols. For example, such an exemplary programming system optionally includes hardware and/or software capable of performing the exemplary analysis 914 of Fig. 9 and/or the exemplary method 1000 of Fig. 10.

While commercially available programming systems such as the Model 3510 allow for automated follow-up protocol and up to 24 customizable automatic follow-up protocols, an exemplary programming system may automatically record procedural information over one or more follow-up consultations, analyze the information and then automatically implement a protocol for a subsequent follow-up consultation or allow a care provider to select manually a recommend protocol or, for example, a customized protocol that is optionally based at least in part on analyzed information.

As described herein, an exemplary programming system may provide an option for care providers to review historical diagnostic data and programming values when necessary wherein such an option may be part of a protocol. Yet further, an exemplary programming system may, for example, after a predetermined number of follow-up consultations or sessions, automatically record patient information, analyze the patient information and then diagnose patient condition. Such an exemplary programming system optionally recommends and/or presents changes to one or more parameters related to an implanted device (e.g., operational parameters that may affect the patient condition).

### Training Care Providers

Procedural information and/or analyzed information may aid in training care providers. According to various training programs a care provider trainee should be capable of managing implanted devices for both bradyarrhythmias and tachyarrhythmias, including both implantation and long term follow-up of such devices. While actual implantation of such devices may not be required, the ability to evaluate an implanted device's performance at implantation frequently is required. Some training programs recommend, for implantable cardioverter-defibrillator devices (ICD), participation in at least 10 implants and ICD device follow-up experience with at least 50 follow-up consultations.

Other training programs required that a trainee participate in at least 100 follow-up consultations with patients having implanted arrhythmia control devices. In such programs, a trainee should be the primary operator and evaluator during the 100 follow-up consultations. A trainee should be able to demonstrate knowledge of an approach to routine follow-up and troubleshooting of implantable devices. Hands-on assessment should include interpretation of paced and non-paced electrocardiograms, interrogation and programming of implanted devices, evaluation of pacemaker dependency and use of digital interval counters. In some programs, a trainee is required to actively participate in diagnosis, prescription and management for 50 patients who require pacemaker implantation.

Various exemplary methods and/or devices described herein optionally facilitate training. For example, an exemplary method that records procedural information and analyzes procedural information may analyze the information in a manner that tracks a trainee's progress. Such a method may recommend additional training to overcome potential or demonstrated weaknesses and recommend less training to overcome potential or demonstrated strengths. Hence, such an exemplary method may make training more efficient. Further, such an exemplary method may allow for improvements to training programs.

Various exemplary methods and/or devices described herein optionally allow for enhanced collection and/or analysis of information germane to regulations or regulatory bodies. For example, a product approval or decision process may rely on collected and/or analyzed procedural information. Further, data integrity may be enhanced by knowledge of procedural information.

## Claims

1. An apparatus comprising: means for recording procedures performed by a care provider during a follow-up consultation with a patient having an implanted device; means for analyzing the procedures; and means for recommending one or more procedures for a subsequent follow-up consultation.

2. Apparatus as claimed in Claim 1, **characterised by** further comprising means for communicating with the implanted device.

3. Apparatus as claimed in Claim 1 or Claim 2, **characterised in that** the means for recording comprises an input device for inputting information to a computer and/or a programming system for the implanted device.

4. Apparatus as claimed in any preceding Claim, **characterised in that** the means for recording records procedures in real-time and/or records event after the follow-up consultation and/or records commands for communicating with the implanted device.

5. Apparatus as claimed in any preceding Claim, **characterised in that** the means for analyzing comprises statistical analysis software.

6. Apparatus as claimed in any preceding Claim, **characterised in that** the means for recommending comprises a programming system for the implanted device and/or a display.

7. A method comprising the steps of: recording procedures performed by a care provider during a follow-up consultation with a patient having an implanted device, wherein at least some of the procedures relate to the implanted device; analyzing the procedures; and based at least in part on the analyzing, recommending one or more procedures for a subsequent follow-up consultation.

8. A method as claimed in Claim 7, **characterised in that** the recording step comprises recording: interrogation procedures for interrogating the implanted device and/or procedures for communicating diagnostic data and/or procedures for threshold assessments and/or procedures for rhythm assessments.

9. A method as claimed in Claim 7 or Claim 8, **characterised in that** the analyzing step comprises engaging in statistical analysis.

10. A method as claimed in Claim 9, **characterised in that** the statistical analysis comprises confidence level analysis.

11. A method as claimed in any of Claims 7 to 10, **characterised in that** the analyzing step comprises: pattern analysis and/or comparing the procedural information to previously recorded procedural information and/or relying at least in part on guidelines.

12. A method as claimed in any of Claims 7 to 11, **characterised in that** the recommending step comprises: recommending one or more procedures for the patient and/or recommending one or more procedures for the care provider and/or recommending one or more procedures for a different patient and/or recommending one or more procedures for patients having an implanted device manufactured by the same manufacturer as the implanted device.

13. A method as claimed in any of Claims 7 to 12, **characterised by** presenting the one or more recommended procedures to a care provider during a subsequent follow-up consultation with the patient having the implanted device.

14. A programming system for an implanted device comprising: an input operative to input procedural information; statistical analysis software operative to analyze the procedural information; and an output operative to output one or more recommended procedures based at least in part on a statistical analysis of the procedural information performed using the statistical analysis software.
